# EUROPEAN PATENT SPECIFICATION

(11) **EP 1 208 393 B1**
(45) Date of publication and mention of the grant of the patent: **08.10.2008**
(21) Application number: 00961359.7
(22) Date of filing: 23.08.2000
(51) Int. Cl.: G02B 1/04, A61L 27/34

(54) **COVALENTLY-BOUND, HYDROPHILIC COATING COMPOSITIONS FOR IMPLANTS**
KOVALENT GEBUNDENE, HYDROPHILE BESCHICHTUNGSZUSAMMENSETZUNGEN FÜR IMPLANTATE
COMPOSITIONS DE REVETEMENT HYDROPHILE LIEES PAR COVALENCE POUR IMPLANTS

(30) Priority: 02.09.1999 US 152507 P
(43) Date of publication of application: 29.05.2002
(60) Divisional application: 06005509.2
(73) Proprietor: Alcon, Inc., 6331 Hünenberg (CH)
(72) Inventor: LEBOEUF, Albert, R., Burleson, TX 76028 (US)
(74) Representative: Keller, Günter
(86) International application number: PCT/US2000/023286
(87) International publication number: WO 2001/016626

(56) References cited:
- WO-A-95/11279
- WO-A-96/40303
- WO-A-99/52570

## Description

### FIELD OF THE INVENTION

This invention relates to coatings for surgical implants. In particular, the present invention relates to hydrophilic, covalently cross-linked copolymers that are covalently bound to the surface of surgical implants.

### BACKGROUND OF THE INVENTION

Both rigid and foldable implantable ophthalmic lens materials are known. The most common rigid material used in ophthalmic implants is polymethyl methacrylate ("PMMA"). Foldable intraocular lens ("IOL") materials can generally be divided into three categories: silicone materials, hydrogel materials, and non-hydrogel ("hydrophobic") acrylic materials. See, for example, Foldable Intraocular Lenses, Ed. Martin et al., Slack Incorporated, Thorofare, New Jersey (1993). For purposes of the present application, hydrophobic acrylic materials are acrylic materials that absorb less than approximately 5% water at room temperature.

Silicone and non-hydrogel acrylic materials used in ophthalmic implants can potentially damage endothelial cells and perhaps other cells or tissues as well during or after the implant's insertion in the eye. These materials are generally hydrophobic and/or tacky and can pull cells off of eye tissues that contact the implant. Particularly in the case of phakic IOL's implanted between the capsular bag and the iris, there is significant potential for physical contact between the implant and surrounding cells or tissue even after the implant reaches its target location.

WO 96/40303 discloses soft, high refractive index, acrylic materials, especially useful as intraocular lens material, containing an aryl acrylic hydrophobic monomer and a hydrophilic monomer.

### SUMMARY OF THE INVENTION

The present invention relates to hydrophilic coating compositions for surgical implants, particularly ophthalmic implants comprising silicone, hydrophobic acrylic or hydrogel materials. More specifically, the present invention relates to a copolymeric coating material for an implant where the copolymeric coating material comprises 2-phenylethyl (meth)acrylate and a hydrophilic monomer selected from the group consisting of hydroxyalkyl (meth)acrylates and acrylamides, a reactive plasticizer selected from the group consisting of polyethylene glycol (200-2000) mono(meth)acrylates; polyethylene glycol (200-2000) monomethylether mono(meth)acrylates; and a crosslinking agent. The coating material is capable of absorbing from about 40 to about 70% water.

The present invention is useful for methods for applying a coating copolymer comprising 2-phenylethyl (meth)acrylate and a hydrophilic monomer as specified above to an implant's surface. The method comprises dissolving the copolymer containing a latent crosslinking agent in a solvent to form a coating solution, contacting the coating solution with the implant's surface, and activating the latent crosslinking agent in the coating copolymer. The method also comprises dissolving the copolymer in a solvent to form a coating solution, adding a crosslinking agent to the coating solution, contacting the coating solution with the implant's surface, and heating the coated implant to generate crosslinking.

The method of applying a coating to a surgical implant thus comprises the steps of
a) preparing an uncrosslinked copolymer comprising 2-phenylethyl (meth)acrylate, a hydrophilic monomer selected from the group consisting of hydroxyalkyl (meth)acrylates; polyethylene glycol (200-500) mono(meth)acrylates; polyethylene glycol (200-500) monomethylether mono(meth)acrylates; and acrylamides, and optionally a latent crosslinking agent, such that the copolymer is capable of absorbing from about 40 to about 70% water;
b) forming a coating solution by dissolving the copolymer in an organic solvent and if the copolymer does not contain a latent crosslinking agent then adding a crosslinking agent to the solution;
c) applying the coating solution to the implant; and
d) drying the coating solution on the implant, such that the latent crosslinking agent or crosslinking agent is activated and the copolymer is covalently bound to the implant.

### DETAILED DESCRIPTION OF THE INVENTION

Unless indicated otherwise, all amounts are expressed as weight %.

The coating material of the present invention is a copolymer comprising 2-phenylethyl (meth)acrylate and a hydrophilic monomer selected from the group consisting of hydroxyalkyl (meth)acrylates and acrylamides, a reactive plasticizer selected from the group consisting of polyethylene glycol (200-2000) mono(meth)acrylates and polyethylene glycol (200-2000) monomethylether mono(meth)acrylates and a crosslinking agent. Preferred hydrophilic monomers are hydroxyalkyl (meth)acrylates. Most preferred are 2-hydroxyethyl methacrylate; 1,3-dihydroxypropyl methacrylate; 2,3-dihydroxypropyl methacrylate; mixtures of 1,3- and 2,3-dihydroxypropyl methacrylate ("GMMA"); monomethoxy glyceryl methacrylate; and mixtures thereof. The most preferred 2-phenylethyl (meth)acrylate is 2-phenylethyl methacrylate ("2-PEMA").

The coating material is capable of absorbing from about 40 to about 70% water, preferably from about 42 to about 55% water. The proportion of the copolymer's monomers will depend on the desired water content, with individual concentrations generally ranging from about 25 to about 60% for 2-phenylethyl (meth)acrylate and about 40 to about 75% for the hydrophilic monomer. In the most preferred embodiment, the desired water content is about 42 to about 55% and the coating material is comprises from 25 to 40% of 2-PEMA and from 25 to 35% of GMMA.

In one embodiment, in addition to the ingredients described above, the coating material also comprises a latent cross-linking agent, such as a blocked isocyanate. Suitable blocked isocyanate compounds include imidazole blocked isocyanatoethyl methacrylate. In this embodiment, the latent cross-linking agent is copolymerized with the other ingredients of the coating copolymer. In an alternative embodiment, the cross-linking agent is not added until the point where the coating copolymer is dissolved to form a coating solution. Examples of cross-linking agents that are suitable for use in this alternative embodiment include di-imidazole blocked 1,12-isocyanatododecane and peroxides, such as benzoyl peroxide and 2,4-dichlorobenzoyl peroxide.

The amount of the cross-linking agent contained in the coating compositions of the present invention will depend upon, among other factors, the chosen cross-linking agent and the degree of cross-linking desired. In general, the amount of the cross-linking agent necessary to cross-link the coating composition and secure it to the implant's surface will be about 0.5 - 3% for blocked isocyanates and about 3 - 6% for peroxides.

The copolymeric coating material is prepared by combining the chosen 2-phenylethyl (meth)acrylate, hydrophilic monomer and a polymerization initiator (optionally with a latent cross-linking agent) to form a coating composition and then curing the coating composition. Any type of polymerization initiator may be used, including thermal initiators and photoinitiators, provided that the initiator can be activated without activating the latent cross-linking agent if present. Preferred initiators are UV- and blue-light activated initiators. The most preferred initiator is the benzoylphosphine oxide initiator, 2,4,6-trimethyl-benzoyldiphenylophosphine oxide ("TPO"), which is activated by blue-light. The amount of the polymerization initiator in the coating compositions of the present invention will depend upon the curing conditions. In general, however, the amount will be about 3 % (w/w) or less, preferably about 2 % (w/w) or less, and most preferably about 1 % (w/w).

In order to prevent premature cross-linking, the coating compositions of the present invention do not contain significant amounts of monomers having more than one unsaturated bond. Such ingredients include the common cross-linking monomers ethyleneglycol dimethacrylate; diethylene glycol dimethacrylate; ethyleneglycol diacrylate; allyl methacrylates; allyl acrylates; 1,3-propanediol dimethacrylate; 1,6-hexanediol dimethacrylate; 1,4-butanediol dimethacrylate; polyethyleneoxide diacrylates; and the like.

In addition to the 2-phenylethyl (meth)acrylate, hydrophilic monomer, any latent cross-linking agent, and polymerization initiator, the coating compositions optionally include one or more ingredients selected from the group consisting of UV absorbers that are copolymerizable with the 2-phenylethyl (meth)acrylate and hydrophilic monomer; blue-light blocking colorants that are copolymerizable with the 2-phenylethyl (meth)acrylate and hydrophilic monomer; reactive plasticizers to minimize haze or crazing; and chain transfer agents to retard cross-linking within the coating copolymer.

Ultraviolet absorbing chromophores can be any compound which absorbs light having a wavelength shorter than about 400 nm, but does not absorb any substantial amount of visible light. Suitable copolymerizable ultraviolet absorbing compounds are the substituted 2-hydroxybenzophenones disclosed in U.S. Patent No. 4,304,895 and the 2-hydroxy-5-acryloxyphenyl-2H-benzotriazoles disclosed in U.S. Patent No. 4,528,311. The most preferred ultraviolet absorbing compound is 2-(3'-methallyl-2'-hydroxy-5'-methyl phenyl) benzotriazole. Suitable polymerizable blue-light blocking chromophores include those disclosed in U.S. Patent No. 5,470,932. If a blue-light activated polymerization initiator is chosen and a blue-light blocking colorant is added, the polymerization initiator identity or concentration may have to be adjusted to minimize any interference.

Suitable reactive plasticizers or softening agents include polyethylene glycol (200 - 2000) mono(meth)acrylates and polyethylene glycol (200 - 2000) monomethylether mono(meth)acrylates. Methacrylates are preferred, with PEG(400)monomethylether monomethacrylate most preferred. If needed or desired, the amount of the reactive plasticizer will range from about 5 to about 25%. Depending on the implant's function and the thickness of the coating, some degree of haze or crazing may be tolerated such that a reactive plasticizer may not be required.

The chain transfer agent, if present, is typically added in an amount ranging from 0.01 to 0.4%. Many chain transfer agents are known in the art. Examples of suitable chain transfer agents include 1-dodecanethiol and 2-mercaptoethanol.

After the coating copolymer is polymerized, a coating solution is prepared by dissolving the coating copolymer in a solvent or mixture of solvents, such as a 50:50 (parts by weight) mixture of ethanol and 2-pentanone. The solvent or mixture of solvents is preferably chosen to give a clear, homogenous coating solution where the chosen solvent or solvent mixture does not evaporate so quickly that it leaves a hazy coating. If no latent cross-linking agent was included in the coating copolymer, a cross-linking agent, such as di-imidazole blocked 1,12-isocyanatododecane or a peroxide, is added to the coating solution.

The concentration of the coating copolymer in the coating solution will depend on the desired coating thickness. Other factors that will influence the thickness of the coating include the viscosity of the coating solution, the temperature of the coating solution and the implant, and the evaporation rate of the chosen solvent(s). In general, the coatings of the present invention will be no more than 1 µm thick, and preferably will be about 0.5 µm thick. A minimum coating thickness of about 0.01 µm is likely necessary to allow the coating to survive any manipulation of the implant (such as the folding of an IOL) and any abrasion caused during implantation or extended residence at the target site in a patient. A concentration of coating copolymer of about 7 - 9 % in the coating solution will typically produce a coating about 0.5 µm thick in a dip-coating process.

The coating solution is applied to the implant by conventional techniques, such as spin- or dip-coating processes. Dip-coating is preferred. The implant is preferably dipped quickly so as to minimize any swelling of the implant caused by the solvent in the coating solution.

After the coating is applied to the implant, the coating is dried. A two-stage drying process is preferred. First, the coated implant is allowed to dry in air until most or all of the solvent has evaporated (generally ≤ 15 minutes). Second, the coated implant is baked at elevated temperature, about 80 - 110 °C, to eliminate as much of the remaining solvent as possible and to activate the cross-linking agent. A preferred drying process involves room temperature air drying for 15 minutes, followed by baking at 100 °C for about 2 hours for blocked isocyanate cross-linking agents and 110 °C for about 3 - 5 hours for peroxide cross-linking agents.

Once the coating is secured to the implant's surface by covalent bonds formed by activating the cross-linking agent, the coating cannot be removed by solvents or solvent mixtures, including the same solvent used as the base in the preparation of the coating solution. After the coating is covalently-bound to the implant's surface, any impurities or unbound ingredients in the coating (or implant) may be removed by extraction in a suitable solvent, such as acetone in the case where the implant is an ACRYSOF^{®} IOL. It is important that the swell response of the coating and the substrate to the extraction solvent not be too different in order to prevent damage to one or both during the extraction process. To prevent crazing or cracking of the coating, the swell of the coating should be greater than or equal to that of the substrate.

Before the coated implant is manipulated, the coating is preferably hydrated for several seconds to minimize crazing or other damage to the coating.

The implants suitable for coating with the hydrophilic coatings of the present invention are preferably made of hydrophobic acrylic materials, but could also be constructed of silicone, silicone-acrylic copolymers or hydrogels. Preferred hydrophobic acrylic materials are those described in U.S. Patent Nos. 5,290,892 and 5,693,095. In the case where the implant is an IOL, the coatings of the present invention may be used in conjunction with substrate materials intended for use as a "hard" IOL (that is inserted in an unfolded state) or a "foldable" or "soft" IOL (that is inserted in a folded or compressed state). For example, the IOL material to be coated could be those IOL materials disclosed in U.S. Patent Nos. 5,693,095 or 5,331,073. The coating may be applied to the entire IOL or to only a portion of the IOL. As used herein, "implants" includes contact lenses.

In order to prepare the implant material to be coated so that it is capable of receiving the coating, it may be necessary or desirable to expose the surface to be coated to a reactive plasma gas prior to applying the coating composition of the present invention. Suitable reactive plasma gases include oxidizing gases, such as oxygen gas. A suitable plasma chamber is the P²CIM B-Series plasma chamber made by Advanced Plasma Systems, Inc. Using such a chamber, suitable plasma parameters include: power = 400 W, plasma gas = oxygen; pressure of the plasma gas = 30 Pa (225 mTorr); exposure time = 4 - 6 minutes.

The following examples are intended to be illustrative but not limiting.

### Examples 1 - 2 (latent cross-linking agent):

The formulations shown in Table 1 below were prepared and cured in polypropylene slab molds (10 mm x 20 mm x 0.9 mm). The formulations were cured by exposure to blue light for one hour using a Kulzer Palatray CU blue light unit (12 - 14 mW/cm²).

### Coating Solution Preparation

The coating copolymer of Example 1 was dissolved in a 21:3:2 (pbw) 2-pentanone:ethanol:dichloromethane solvent to give an 8.4% solution. The coating copolymer of Example 2 was dissolved in 2-pentanone to give an 8.0% solution. The resulting solutions were filtered through a Gelman glass fiber Acrodisc (1 µm) to give particulate-free coating solutions.

### Coating Application

A copolymer comprising 65% 2-phenylethyl acrylate; 30% 2-phenylethyl methacrylate; 1.8% o-methyl Tinuvin P; and 3.2% 1,4-butanediol diacrylate was prepared using 1.8% Perkadox-16 as a thermal initiator. This copolymer was cured in the slab molds described above, extracted in acetone for approximately 2 hours, dried in air at room temperature for about 1 hour, and then dried in an oven at 100 °C for about 1 hour. This material in the form of the defined slabs served as the implant/substrate material for all Examples ("the implant slabs").

The implant slabs were dipped in the coating solutions. Caution is taken to minimize the immersion time of the samples in the coating solution as the solvent will swell the sample. The coated implant was allowed to dry in air at room temperature for 15 minutes, followed by baking at 100 °C for 2 hours to activate the latent cross-linking agent and secure the coating to the implant's surface.

**TABLE 1**

| (all amounts in parts by weight) | | |
|---|---|---|
| INGREDIENT | 1 | 2 |
| 2-PEMA | 38.93 | 28.31 |
| 2-HEMA | --- | 34.66 |
| GMMA | 29.25 | --- |
| PEG (400) Monomethylether Monomethacrylate | 29.45 | 34.93 |
| Imidazole Blocked Isocyanatoethyl Methacrylate | 1.05 | 1.0 |
| 1-Dodecanethiol | 0.41 | 0.30 |
| Lucirin TPO | 0.89 | 0.78 |
| t-Butylperoctoate | | |
| | | |
| % water (slab) | 52.5 | 44.6 |
| Refractive Index (hydrated) | 1.425 | 1.429 |

### Example 3 (cross-linking agent added when coating solution is formed):

Coated implant slabs are prepared according to the procedure described above for Examples 1 - 2, except that the coating copolymer contains the ingredients shown in Table 2 below. The coating copolymer is cured using the Kulzer Palatray CU unit for one hour. A coating solution is formed by dissolving the coating copolymer in 2-pentanone to form an 8.0% solution. The implant slabs are dip coated and allowed to dry in air at room temperature for 15 minutes, followed by baking at 100 °C for 2 hours to de-block the isocyanate cross-linking agent and secure the coating to the implant's surface.

**TABLE 2**

| (all amounts in parts by weight) | |
|---|---|
| INGREDIENT | 3 |
| 2-PEMA | 28.31 |
| 2-HEMA | 34.66 |
| GMMA | --- |
| PEG (400) Monomethylether Monomethacrylate | 34.93 |
| di-Imidazole Blocked 1,12-diisocyanatododecane | 1.0 |
| 1-Dodecanethiol | 0.30 |
| Lucirin TPO | 0.78 |
| t-Butylperoctoate | |
| | |
| % water (slab) | 44.6 |
| Refractive Index (hydrated) | 1.429 |

The invention has been described by reference to certain preferred embodiments. The embodiments described above are therefore considered to be illustrative in all respects and not restrictive, the scope of the invention being indicated by the appended claims.

## Claims

1. A copolymeric coating composition for a surgical implant wherein the coating composition comprises 2-phenylethyl (meth)acrylate, a hydrophilic monomer selected from the group consisting of hydroxyalkyl (meth)acrylates and acrylamides, a reactive plasticizer selected from the group consisting of polyethylene glycol (200 - 2000) mono(meth)acrylates and polyethylene glycol (200 - 2000) monomethylether mono(meth)acrylates, and a cross-linking agent, and wherein the coating composition is capable of absorbing from about 40 to about 70% water.

2. The coating composition of Claim 1 wherein the hydrophilic monomer is a hydroxyalkyl (meth)acrylate.

3. The coating composition of Claim 2 wherein the 2-phenylethyl (meth)acrylate is 2-phenylethyl methacrylate and the hydrophilic monomer is selected from the group consisting of 2-hydroxyethyl methacrylate; 1,3-dihydroxypropyl methacrylate; 2,3-dihydroxypropyl methacrylate; mixtures of 1,3- and 2,3-dihydroxypropyl methacrylate; monomethoxy glyceryl methacrylate; and mixtures thereof.

4. The coating composition of Claim 3 wherein the hydrophilic monomer is a mixture of 1,3- and 2,3-dihydroxypropyl methacrylate.

5. The coating composition of Claim 1 wherein the coating composition is capable of absorbing from about 42 to about 55% water.

6. The coating composition of Claim 1 wherein the coating composition comprises from about 25 to about 60% of 2-phenylethyl (meth)acrylate and from about 40 to about 75% of the hydrophilic monomer.

7. The coating composition of Claim 6 wherein the coating composition comprises from 25 to 40% of 2-phenylethyl (meth)acrylate and from 25 to 35% of the hydrophilic monomer.

8. The coating composition of Claim 1 wherein the cross-linking agent is selected from the group consisting of blocked isocyanates and peroxides.

9. The coating composition of Claim 8 wherein the cross-linking agent is selected from the group consisting of imidazole blocked isocyanatoethyl methacrylate; di-imidazole blocked 1,12-isocyanatododecane; benzoyl peroxide; and 2,4-dichlorobenzoyl peroxide.

10. The coating composition of Claim 1 wherein the coating composition further comprises one or more-ingredients selected from the group consisting of UV absorbers that are copolymerizable with the 2-phenylethyl (meth)acrylate and hydrophilic monomer; blue-light blocking colorants that are copolymerizable with the 2-phenylethyl (meth)acrylate and hydrophilic monomer; reactive plasticizers; and chain transfer agents.

11. The coating composition of Claim 1 wherein the reactive plasticizer is polyethylene glycol (400) monomethylether monomethacrylate.

12. The coating composition of Claim 10 wherein the coating composition comprises a chain transfer agent selected from the group consisting of 1-dodecanethiol and 2-mercaptoethanol.

## Patentansprüche

1. Copolymer-Beschichtungszusammensetzung für ein chirurgisches Implantat, wobei die Beschichtungszusammensetzung 2-Phenylethyl(meth)acrylat, ein hydrophiles Monomer, ausgewählt aus der Gruppe, bestehend aus Hydroxyalkyl(meth)acrylaten und Acrylamiden, einen reaktiven Weichmacher, ausgewählt aus der Gruppe, bestehend aus Polyethylenglycol(200-2000)mono(meth)acrylaten und Polyethylenglycol(200-2000)monomethylethermono(meth)-acrylaten, und ein Vernetzungsmittel umfaßt, und wobei die Beschichtungszusammensetzung etwa 40 bis etwa 70 % Wasser absorbieren kann.

2. Beschichtungszusammensetzung nach Anspruch 1, wobei das hydrophile Monomer ein Hydroxyalkyl(meth)acrylat ist.

3. Beschichtungszusammensetzung nach Anspruch 2, wobei das 2-Phenylethyl(meth)acrylat 2-Phenylethylmethacrylat ist und das hydrophile Monomer aus der Gruppe ausgewählt ist, bestehend aus 2-Hydroxyethylmethacrylat; 1,3-Dihydroxypropylmethacrylat; 2,3-Dihydroxypropylmethacrylat; Gemischen aus 1,3- und 2,3-Dihydroxypropylmethacrylat; Monomethoxyglycerylmethacrylat und Gemischen davon.

4. Beschichtungszusammensetzung nach Anspruch 3, wobei das hydrophile Monomer ein Gemisch aus 1,3- und 2,3-Dihydroxypropylmethacrylat ist.

5. Beschichtungszusammensetzung nach Anspruch 1, wobei die Beschichtungszusammensetzung etwa 42 bis etwa 55 % Wasser absorbieren kann.

6. Beschichtungszusammensetzung nach Anspruch 1, wobei die Beschichtungszusammensetzung etwa 25 bis etwa 60 % 2-Phenylethyl(meth)acrylat und etwa 40 bis etwa 75 % des hydrophilen Monomers umfaßt.

7. Beschichtungszusammensetzung nach Anspruch 6, wobei die Beschichtungszusammensetzung 25 bis 40 % 2-Phenylethyl(meth)acrylat und 25 bis 35 % des hydrophilen Monomers umfaßt.

8. Beschichtungszusammensetzung nach Anspruch 1, wobei das Vernetzungsmittel aus der Gruppe ausgewählt ist, bestehend aus blockierten Isocyanaten und Peroxiden.

9. Beschichtungszusammensetzung nach Anspruch 8, wobei das Vernetzungsmittel aus der Gruppe ausgewählt ist, bestehend aus mit Imidazol blockiertem Isocyanatoethylmethacrylat; mit Diimidazol blockiertem 1,12-Isocyanatododecan; Benzoylperoxid und 2,4-Dichlorbenzoylperoxid.

10. Beschichtungszusammensetzung nach Anspruch 1, wobei die Beschichtungszusammensetzung ferner einen oder mehrere Bestandteile, ausgewählt aus der Gruppe, bestehend aus UV-Absorbern, die mit dem 2-Phenylethyl(meth)acrylat und dem hydrophilen Monomer copolymerisierbar sind; Blaulicht blockierenden Färbemitteln, die mit dem 2-Phenylethyl-(meth)acrylat und dem hydrophilen Monomer copolymerisierbar sind; reaktiven Weichmachern und Kettenüberträgern, umfaßt.

11. Beschichtungszusammensetzung nach Anspruch 1, wobei der reaktive Weichmacher Polyethylenglycol(400)monomethylethermonomethacrylat ist.

12. Beschichtungszusammensetzung nach Anspruch 10, wobei die Beschichtungszusammensetzung einen Kettenüberträger, ausgewählt aus der Gruppe, bestehend aus 1-Dodecanthiol und 2-Mercaptoethanol, umfaßt.

## Revendications

1. Composition de revêtement copolymérique pour implant chirurgical dans laquelle la composition de revêtement comprend du 2-phényléthyl (méth)acrylate, un monomère hydrophile choisi dans le groupe comprenant les hydroxyalkyl (méth)acrylates et acrylamides, un plastifiant réactif choisi dans le groupe comprenant les mono(méth)-acrylates de polyéthylène glycol (200-2000) et les mono(méth)acrylates de monométhyléther de polyéthylène glycol (200-2000) et un agent de réticulation, et dans laquelle la composition de revêtement peut absorber environ 40 à environ 70 % d'eau.

2. Composition de revêtement suivant la revendication 1, dans laquelle le monomère hydrophile est un hydroxyalkyl (méth)-acrylate.

3. Composition de revêtement suivant la revendication 2, dans laquelle le 2-phényléthyl (méth)acrylate est du 2-phényléthyl (méth)acrylate et le monomère hydrophile est choisi dans le groupe comprenant le 2-hydroxyéthyl méthacrylate, le 1,3-dihydroxypropyl méthacrylate, le 2,3-dihydroxypropyl méthacrylate, les mélanges de 1,3- et 2,3-dihydroxypropyl méthacrylate, le monométhoxy glycéryl méthacrylate et leurs mélanges.

4. Composition de revêtement suivant la revendication 3, dans laquelle le monomère hydrophile est un mélange de 1,3- et 2,3-dihydroxypropyl méthacrylate.

5. Composition de revêtement suivant la revendication 1, dans laquelle la composition de revêtement peut absorber environ 42 à environ 55 % d'eau.

6. Composition de revêtement suivant la revendication 1, dans laquelle la composition de revêtement comprend environ 25 à environ 60 % de 2-phényléthyl (méth)acrylate et environ 40 à environ 75 % du monomère hydrophile.

7. Composition de revêtement suivant la revendication 6, dans laquelle la composition de revêtement comprend de 25 à 40 % de 2-phényléthyl (méth)acrylate et de 25 à 35 % du monomère hydrophile.

8. Composition de revêtement suivant la revendication 1, dans laquelle l'agent de réticulation est choisi dans le groupe comprenant les isocyanates bloqués et les peroxydes.

9. Composition de revêtement suivant la revendication 8, dans laquelle l'agent de réticulation est choisi dans le groupe comprenant l'isocyanatoéthyl méthacrylate bloqué à l'imidazole, le 1,12-iso-cyanatododécane bloqué au diimidazole, le peroxyde de benzoyle et le peroxyde de 2,4-dichlorobenzoyle.

10. Composition de revêtement suivant la revendication 1, dans laquelle la composition de revêtement comprend de plus un ou plusieurs ingrédients choisis dans le groupe comprenant les absorbeurs d'U.V. qui sont copolymérisables avec le 2-phényléthyl (méth)acrylate et le monomère hydrophile, les colorants bloquant la lumière bleue qui sont copolymérisables avec le 2-phényléthyl (méth)acrylate et le monomère hydrophile, les plastifiants réactifs et les agents de transfert de chaîne.

11. Composition de revêtement suivant la revendication 1, dans laquelle le plastifiant réactif est du monométhacrylate de monométhyléther de polyéthylène glycol (400).

12. Composition de revêtement suivant la revendication 10, dans laquelle la composition de revêtement comprend un agent de transfert de chaîne choisi dans le groupe comprenant le 1-dodécane-thiol et le 2-mercaptoéthanol.
